# EUROPEAN PATENT SPECIFICATION

(11) **EP 0 288 307 B1**
(45) Date of publication and mention of the grant of the patent: **04.09.1996**
(21) Application number: 88303658.4
(22) Date of filing: 22.04.1988
(51) Int. Cl.: C12N 15/81, C12N 1/16, C12N 5/00, A61K 38/22

(54) **Recombinant production of PDGF A-chain polypeptides**
Rekombinante Herstellung von A-Ketten-Polypeptiden von PDGF
Préparation par recombinaison de polypeptides de la chaîne A de PDGF

(30) Priority: 22.04.1987 US 41299
(43) Date of publication of application: 26.10.1988
(60) Divisional application: 95105415.4
(73) Proprietor: CHIRON CORPORATION, Emeryville, California 94608 (US)
(72) Inventor: Heldin, Carl-Henrik, Biomedical Center S-75123 Uppsala (SE); Betsholtz, Christer, S-75185 Uppsala (SE); Westermark, Bengt, S-75185 Uppsala (SE); Knott, Timothy J., London W4 3NJ (GB); Scott, James, c/o MRC Clinical Research Centre, Watford Road Harrow Middlesex HA1 3UJ (GB); Bell, Graeme I., San Francisco, CA 94131 (US); Rall, Leslie, San Francisco, CA 94131 (US)
(74) Representative: Goldin, Douglas Michael

(56) References cited:
- EP-A- 0 177 957
- EP-A- 0 259 632
- NATURE, vol. 320, no. 6064, April 1986, London (GB); C. BETSHOLTZ et al., pp. 695-699
- JOURNAL OF CELL BIOCHEMISTRY, suppL., vol. 0, no. 11, 1987; B.D. Tong et al., p. 44, part A 175
- NATURE, vol. 328, 13 August 1987; B.D. TONG et al., pp. 619-624; and T. COLLINS et al., pp. 621-624

## Description

### Technical Field

The invention is in the fields of biochemistry, molecular biology, and genetic engineering. More particularly it relates to recombinant vectors for cloning and expressing genes for human platelet-derived growth factor (PDGF) A-chain polypeptides and precursor polypeptides in yeast, and methods for producing recombinant PDGF A-chain polypeptides in yeast.

### Background

PDGF is the major mitogen in serum for mesenchymal-derived cells. PDGF is stored in platelet α-granules and released locally during platelet activation when blood vessels are injured. PDGF is a potent chemoattractant for monocytes and neutrophils and for fibroblasts and smooth muscle cells. These activities make PDGF an important component in tissue repair processes.

Purified native PDGF is a glycoprotein of approximately 30,000 daltons and is composed of- two disulfide-linked chains. There are two types of_chains, designated A and B. Whether native PDGF is a heterodimer, a mixture of homodimers or a mixture of heterodimer and homodimer(s) is not known, but the dimer structure is functionally important, since reduction irreversibly destroys the biological activity of PDGF.

The B-chain is derived by proteolytic processing of a 241 amino acid precursor. The B-chain precursor is encoded by the c-sis gene, the cellular counterpart to the transforming gene v-sis of simian sarcoma virus (SSV). cDNA encoding the B-chain has been reported previously in Nature (1985) 316:748-750. There is homology between the B-chain and the transforming protein v-sis. The cloning and expression of the v-sis gene is described in EPA 85112852.0 (publication no. 0177957). Studies of v-sis indicate that B-chain homodimers have mitogenic activity. Also, sequencing of porcine PDGF has revealed that it contains only one type of chain, corresponding to human B-chain (EMBO J (1984) 3:2963-2967).

Johnsson, A., et al, EMBO J (1984) 3:921-928 describe a partial amino acid sequence for PDGF A-chain. See also Nature (1983) 304: 35-39 and Science (1983) 221: 275-277. Heldin, C.H. et al, Nature (1986) 319:511514 describes an osteosarcoma-derived growth factor (ODGF) that is structurally related to putative PDGF A-chain homodimer. The studies of ODGF suggest that PDGF A-chain homodimer would exhibit biological activity. Betsholtz et al describe a cDNA sequence of human PDGF A-chain.

### Disclosure of Invention

The present invention is based on the preparation of vectors for expressing PDGF A-chain polypeptides, and the expression of biologically active PDGF A-chain proteins using such expression vectors.

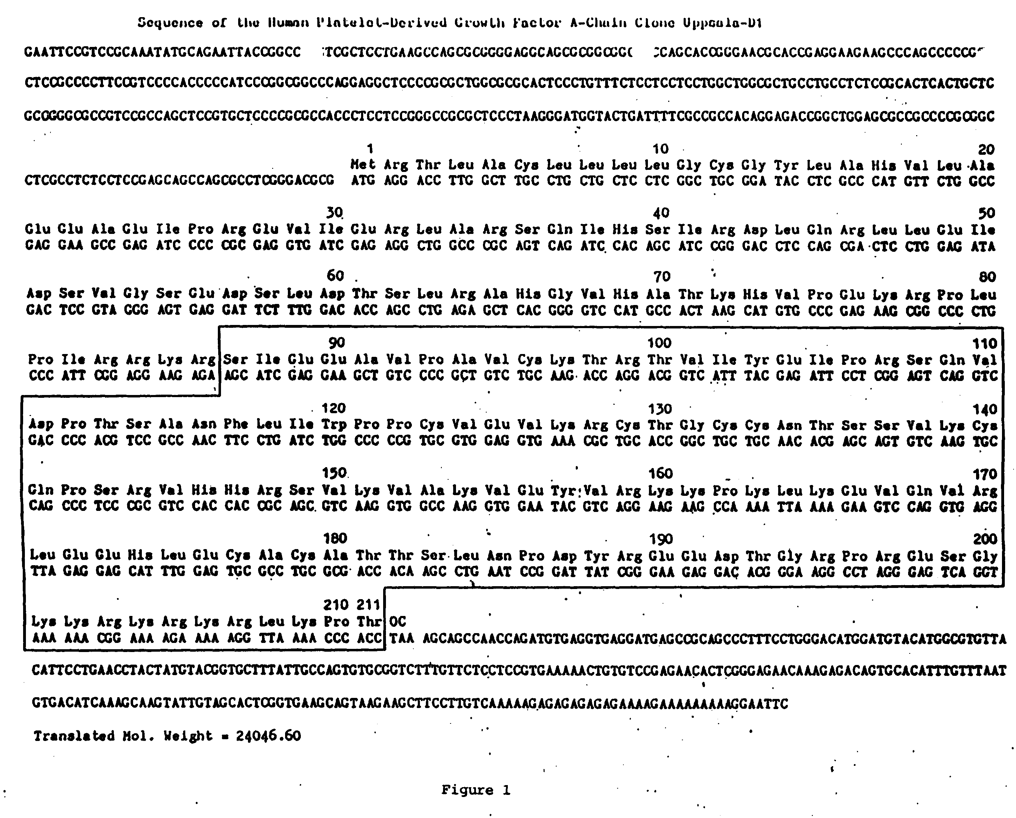

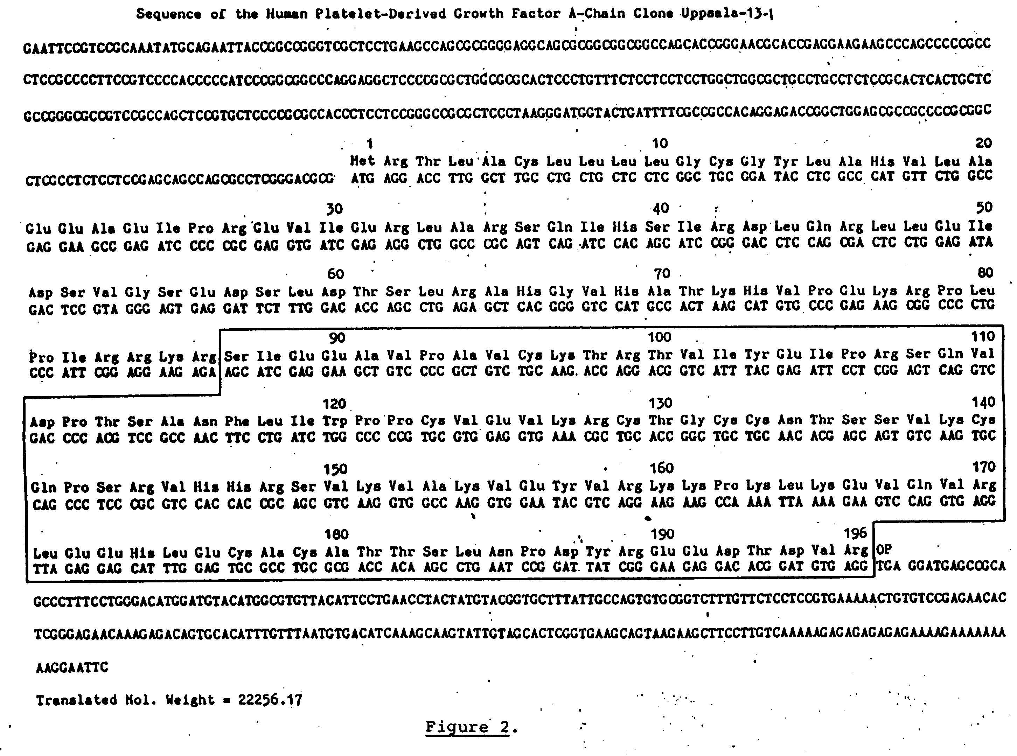

The present invention thus provides a recombinant yeast expression vector comprising:
(i) a DNA sequence which encodes a PDGF A-chain polypeptide comprising the amino acid sequence numbered 87 to 193, inclusive, in Figure 1;
(ii) a DNA sequence which encodes a PDGF A-chain polypeptide comprising the amino acid sequence numbered 87 to 196, inclusive, in Figure 1 or Figure 2;
(iii) a DNA sequence which encodes a PDGF A-chain polypeptide comprising the amino acid sequence numbered 87 to 211, inclusive, in Figure 1;
(iv) a DNA sequence which encodes a PDGF A-chain polypeptide comprising the amino acid sequence numbered 1 to 196, inclusive, in Figure 1 or Figure 2; or
(v) a DNA sequence which encodes a PDGF A-chain polypeptide comprising the amino acid sequence numbered 1 to 211, inclusive, in Figure 1;
operably linked to a yeast hybrid ADH-GAPDH promoter.

Transformed yeast cells which contain such expression vectors and are capable of producing biologically active (as measured by the assay described in §5 of the examples) recombinant PDGF comprised of PDGF A-chain polypeptides are another aspect of the invention.

Methods for producing biologically active PDGF A-chain proteins which employ such hosts are still another aspect of the invention.

Recombinant PDGF comprised of PDGF A-chain polypeptide obtained by growing yeast cells of the invention is a further aspect of the invention.

### Brief Description of the Drawings

Figure 1 shows the nucleotide sequence and deduced amino acid sequence of one form of PDGF A-chain precursor (designated D1).

Figure 2 shows the nucleotide sequence and deduced amino acid sequence of a second form of PDGF A-chain precursor (designated 13-1).

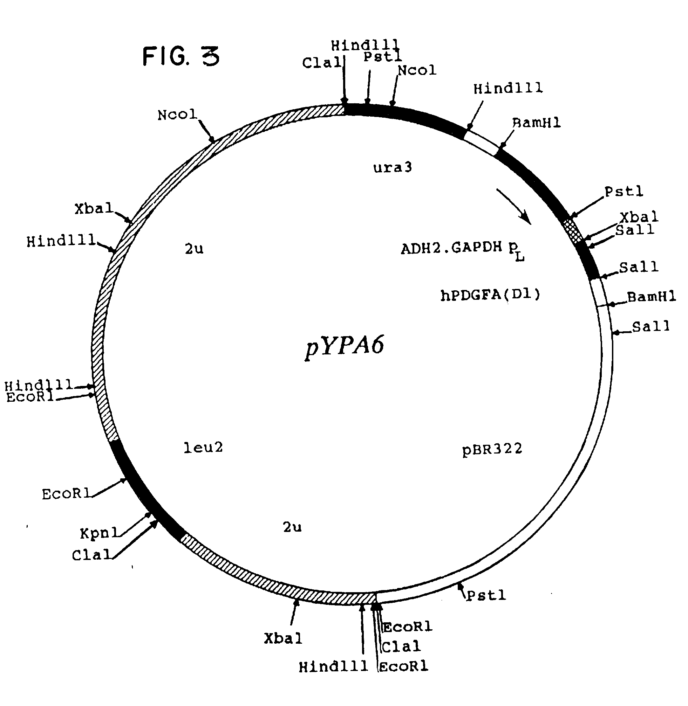

Figure 3 is a map of the plasmid pYpA6 described in the examples (§3.1 .2).

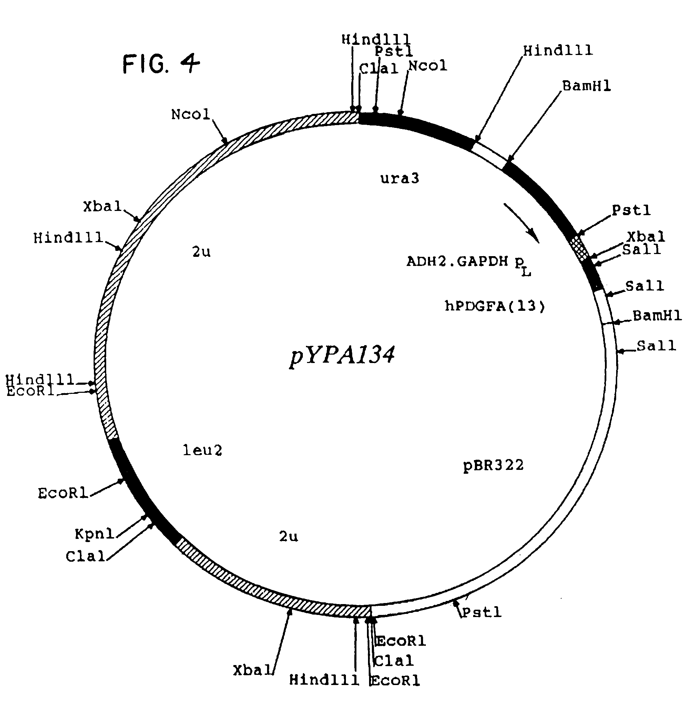

Figure 4 is a map of the plasmid pYpA134 described in the examples (§3.1.2).

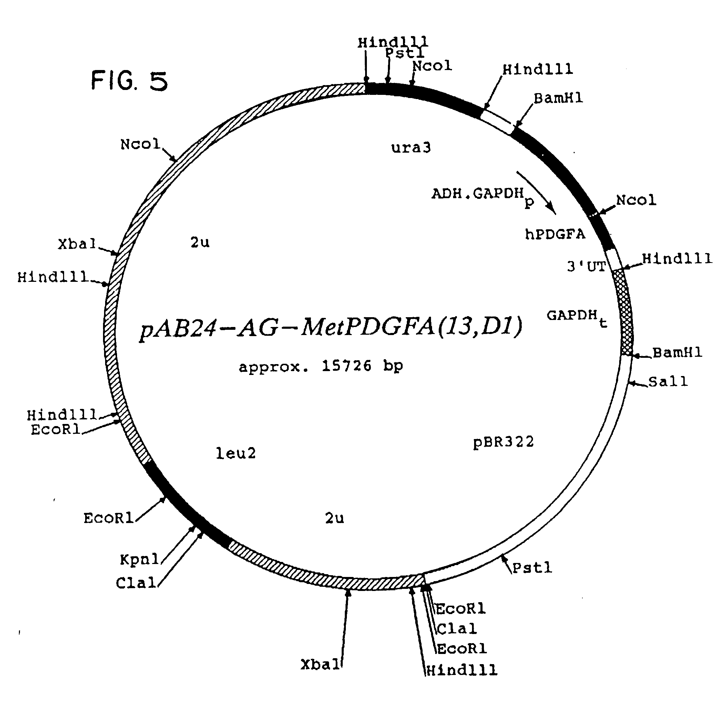

Figure 5 is a map of the plasmids pAB24AGMetPDGFAD1/A13.1 described in the examples (§3.2.2).

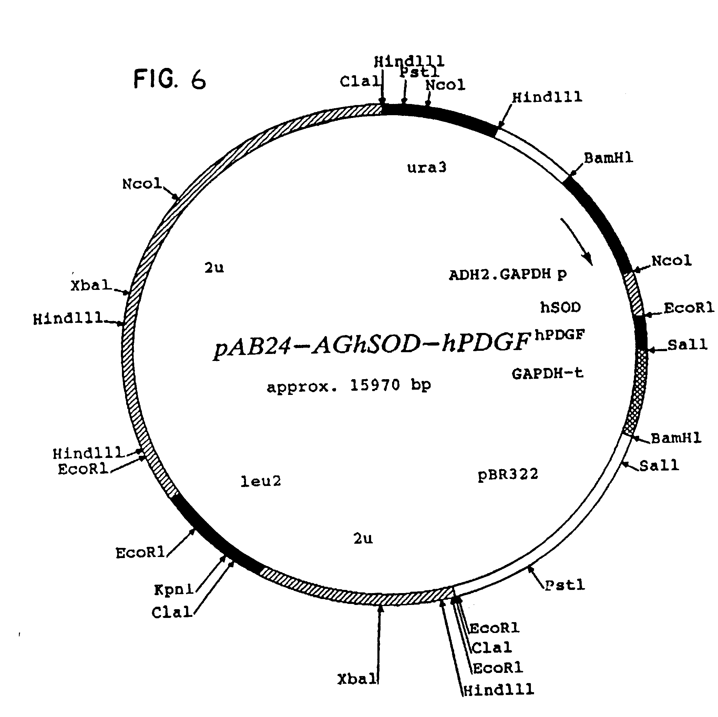

Figure 6 is a map of the plasmids pAB24AGhSODhPDGF AD1/13.1 described in the examples (§3.3.2).

### Modes for Carryinq Out the Invention

### 1. Definitions

The term "recombinant" as used herein to characterize DNA encoding PDGF A-chain polypeptides intends DNA of genomic, cDNA, semisynthetic, or synthetic origin which, by virtue of its origin or manipulation is (1) not associated with all or a portion of the DNA with which it is associated in nature or in the form of a library and/or (2) linked to DNA other than that to which it is linked in nature.

A "replicon" is any genetic element (e.g., a plasmid, a chromosome, a virus) that behaves as an autonomous unit of polynucleotide replication within a cell; i e., capable of replication under its own control.

A "vector" is a replicon in which another polynucleotide segment is attached, so as to bring about the replication and/or expression of the attached segment. An "expression vector" refers to a vector capable of autonomous replication or integration and contains control sequences which direct the transcription and translation of the PDGF A-chain DNA in an appropriate host.

A "coding sequence" is a polynucleotide sequence which is transcribed and/or translated into a polypeptide.

A "promoter sequence" is a DNA regulatory region capable of binding RNA polymerase and initiating transcription of a downstream (i.e., in the 3′ direction) coding sequence.

A coding sequence is "under the control" of the promoter sequence in a cell when transcription of the coding sequence results from the binding of RNA polymerase to the promoter sequence; translation of the resulting mRNA then results in the polypeptide encoded within the coding sequence.

"Operably linked" refers to a juxtaposition wherein the components are configured so as to perform their usual function. Thus, control sequences operably linked to a coding sequence are capable of effecting the expression of the coding sequence.

"Control sequences" refers to those sequences which control the transcription and/or translation of the coding sequence(s); these may include, but are not limited to, promoter sequences, transcriptional initiation and termination sequences, and translational intitiation and termination sequences. In addition, "control sequences" refers to sequences which control the processing of the polypeptide encoded within the coding sequence; these may include, but are not limited to sequences controlling secretion, protease cleavage, and glycosylation of the polypeptide.

"Transformation" is the insertion of an exogenous polynucleotide into a host cell. The exogenous polynucleotide may be maintained as a plasmid, or alternatively, may be integrated within the host genome.

### 2. Recombinant PDGF A-chain DNA

The recombinant PDGF A-chain DNA of the invention encodes at least amino acids 87-193, inclusive of the boxed amino acid sequence shown in Figure 1 and analogs of that amino acid sequence which are substantially homologous and functionally equivalent thereto. The term "substantially homologous" intends that the number of amino acid variations (including substitutions and/or deletions) in said sequence be less than about 10, preferably less than about 3. The term "functionally equivalent" intends that the sequence of the analog defines a chain that will produce a protein having the biological activity of PDGF (as measured by the assay described in §5 of the examples). The DNA may include in addition DNA encoding one or more of amino acid residues 194-196, inclusive of the boxed sequences of Figures 1 or 2, DNA encoding one or more of amino acid residues 197-211, inclusive, of the boxed sequence shown in Figure 1 and/or DNA encoding all or a portion of amino acids 1-86, inclusive of the boxed amino acid sequence shown in Figure 1. A preferred DNA sequence encoding said amino acids 87-193 for expression in mammalian systems is the DNA sequence shown in Figure 1. For expression in other organisms, it may be desirable to use sequences that employ codons preferred by the particular host in which the DNA is expressed.

The recombinant PDGF A-chain DNA may be genomic, cDNA or synthetic DNA. By way of example, the sequences shown in Figures 1 and 2 were obtained from a cDNA library prepared from mRNA of a PDGF-producing cell line. The library was probed with two probes having sequences based on the reported partial amino acid sequence of PDGF A-chain. Clones that hybridized to both probes provided the illustrated sequences. Those sequences may be used to probe human genomic libraries to obtain analogous genomic DNA encoding PDGF A-chain polypeptides. Based on the amino acid sequence deduced from the illustrated sequences, synthetic genes encoding PDGF A-chain polypeptides may be prepared in vitro by synthesizing individual overlapping complementary oligonucleotides and filling in single stranded nonoverlapping portions using DNA polymerase in the presence of the deoxyribonucleotide triphosphates.

The deduced amino acid sequence shown in Figure 1 differs from the reported partial amino acid sequence derived by amino acid sequencing PDGF A-chain at amino acids 119, 141 and 143. The reported residues at those positions were assigned Val, Arg and Thr, respectively. As shown in Figure 1, the PDGF A-chain cDNA indicates these residues are instead Ile, Gln, and Ser, respectively.

### 3. Cloning of PDGF A-chain DNA

The PDGF A-chain DNA can be cloned into any suitable replicon to create a vector, and thereby be maintained in a composition which is substantially free of vectors that do not contain the PDGF A-chain gene (e.g., other clones derived from the library). Numerous cloning vectors are known to those of skill in the art, and the selection of an appropriate cloning vector is a matter of choice. Examples of vectors for cloning and host cells which they can transform include the bacteriophage λ (E. coli), pBR 322 (E. coli), pACYC 177 (E. coli), pKT 230 (gram-negative bacteria), pGV1106 (gram-negative bacteria), pLAFR1 (gram-negative bacteria), pME290 (non-E. coli gram-negative bacteria), pHV 14 (E. coli and Bacillus subtilis), pBD9 (Bacillus), pIJ61 (Streptomyces), pUC6 (Streptomyces), actinophage φC31 (Streptomyces, YIp5 (Saccharomyces, YCp19 (Saccharomyces, and bovine papilloma virus (mammalian cells).

### 4. Expression of PDGF A-Chain DNA

The polynucleotide sequence encoding the PDGF A-chain polypeptide is expressed by inserting the sequence into an appropriate replicon thereby creating an expression vector, and introducing the resulting expression vector into a compatible host.

In creating an expression vector the sequence encoding the PDGF A-chain polypeptide is located in the vector with the appropriate controls sequences. The positioning and orientation of the coding sequence with respect to the control sequences is such that the coding sequence is transcribed under the control of the control sequences: i.e., the promoter will control the transcription of the mRNA derived from the coding sequence; and the ribosomes will bind at the ribosomal binding site to begin the translational process; and the stop codon used to terminate translation will be upstream from the transcriptional termination codon. Control sequences for yeast vectors include promoters for the synthesis of glycolytic enzymes (Hess et al, J Adv Enzyme Reg (1968) 7:149; Holland et al, Biochemistry (1978) 17:4900). Additional promoters known in the art include the promoter for 3-phosphoglycerate kinase (Hitzeman et al, J Biol Chem (1980) 255:2073). Other promoters, which have the additional advantage of transcription controlled by growth conditions and/or genetic background are the promoter regions for alcohol dehydrogenase 2 (ADH2), isocytochrome C, acid phosphatase, degradative enzymes associated with nitrogen metabolism, the alpha factor system and enzymes responsible for maltose and galactose utilization. It is also believed terminator sequences are desirable at the 3' end of the coding sequences. Such terminators are found in the 3' untranslated region following the coding sequences in yeast-derived genes.

In addition to control sequences, it may be desirable to add regulatory sequences which allow for regulation of the expression of the PDGF A-chain gene relative to the growth of the host cell. Examples of regulatory systems are those which cause the expression of a gene to be turned on or off in response to a chemical or physical stimulus, including the presence of a regulatory compound. In eukaryotic systems induction can occur in methallothionein genes with heavy metals and the mouse mammary tumor virus (MMTV) system with steroids. In these cases, the sequence encoding the PDGF A-chain polypeptides would be placed in tandem with the regulatory element.

There are also selective elements which give rise to DNA amplification which in turn can result in higher levels of specific protein production. In eukaryotic systems these include the dihydrofolate reductase gene (dhfr) which is amplified in the presence of methotrexate, and adenosine deaminase (ADA) in the presence of deoxycorfomycin. In these cases the sequence encoding the PDGF A-chain polypeptides may either be present on the same plasmid or merely be cotransfected together with the selectable element to allow for integration within the host cell genome near each other.

Other types of regulatory elements may also be present in the vector, i.e., those which are not necessarily in tandem with the sequence encoding PDGF A-chain. An example is the SV40 enhancer sequence, which, by its mere presence, causes an enhancement of expression of genes distal to it.

Modification of the sequence encoding PDGf A-chain, prior to its insertion into the replicon, may be desirable or necessary, depending upon the expression system chosen. For example, in some cases, it may be necessary to modify the sequence so that it may be attached to the control sequences with the appropriate orientation, i.e., to maintain the reading frame. In some cases, it may be desirable to add sequences which cause the secretion of the polypeptide from the host organism, with subsequent cleavage of the secretory signal. The techniques for modifying nucleotide sequences utilizing cloning are well known to those skilled in the art. They include, e.g., the use of restriction enzymes, of enzymes such as Bal31 to remove excess nucleotides, and of chemically synthesized oligonucleotides for use as adapters, to replace lost nucleotides, and in site directed mutagenesis.

The appropriately modified sequence encoding the PDGF A-chain polypeptide may be ligated to the control sequences prior to insertion into a vector. Alternatively, the coding sequence can be cloned directly into an expression vector which already contains the control sequences and an appropriate restriction site. For expression of the PDGF A-chain polypeptide in yeast, the control sequences will necessarily be heterologous to the coding sequence.

Depending on the host cell used, transformation is done using standard techniques appropriate to such cells. Transformations into yeast may be carried out according to the method of Beggs, J.D., Nature (1978) 275:104-109 or of Hinnen, A., et al, Proc Natl Acad Sci (USA) (1978) 75:1929.

Transformed cells are then grown under conditions which permit expression of the PDGF A-chain gene and assembly of the expression product into a biologically active PDGF (i.e., into a dimeric form). As shown in the following experimental section, initial attempts to produce recombinant PDGF A-chain homodimers in bacteria produced little, if any, biologically active PDGF. This may be due to a variety of reasons, such as improper dimer formation or improper folding. In addition to producing recombinant PDGF A-chain homodimer, the present invention permits production of heterodimers of PDGF A-chain and PDGF B-chain by coexpressing the genes for both PDGF A-chain and PDGF B-chain through use of separate vectors or a single vector that contains an A-chain gene and the B-chain gene. The thus synthesized recombinant PDGF protein is then isolated from the host cells and purified. If the expression system secretes the PDGF into the growth media, the PDGF is isolated directly from the media. If the recombinant PDGF is not secreted, it is isolated from cell lysates. The selection of the appropriate growth conditions and recovery methods are within the skill of the art. With regard to purification, see for instance, EPA Publication No. 0177957 and Nature (1986) 319:511-514.

### Use and Administration of Recombinant PDGF

Recombinant PDGF prepared according to the invention is generally applied topically to wounds such as cutaneous, dermal, mucosal, or epithelial wounds in vertebrates, particularly mammals including man, domestic and farm animals, sports animals and pets. It may be used to treat any type of full or partial thickness wounds including traumatic wounds, surgical wounds, thermal or chemical wounds (burns) radiation wounds, and ulcers such as decubiti and cutaneous ulcers caused by vascular, hematologic and metabolic diseases, infections, or neoplasms.

The PDGF may be formulated using available excipients and carriers in the form of a lotion, spray, gel, ointment or as a controlled or sustained release dosage form. Additional ingredients such as other growth factors (FGF, CTAP-III, EGF, IGF-1, IGF-2, TGF-β, TGF-α), buffers, local anesthetics, antibiotics, gelling agents, and the like may be included in the formulation.

For topical administration, which is the most appropriate with regard to cutaneous lesions, standard topical formulations are employed using, for example, 0.1-10% concentrations of PDGF. The concentration of formulation depends, of course, on the severity of the wound and nature of the subject. In some treatment regimens, the dose is lowered with time to lessen likelihood of scarring.

Controlled or sustained release formulations of recombinant PDGF are made by incorporating the PDGF in carriers or vehicles such as liposomes, nonresorbable semipermeable polymers such as ethylene-vinyl acetate copolymers and Hytrel® copolymers, swellable polymers such as hydrogels, or resorbable polymers such as collagen and certain polyacids or polyesters such as those used to make resorbable sutures to provide for sustained release of the PDGF to the wound site over an extended time period, typically from one day to one week. Such incorporation may be particularly desirable when the PDGF is incorporated into a wound dressing. The mechanism of PDGF release from the formulation may be diffusion, osmosis, leaching, dissolution, erosion, or combinations thereof. In diffusional sustained release formulations the PDGF dissolves in and diffuses through the carrier or vehicle in which it is encapsulated/dispersed. In leaching or dissolution formulations, the PDGF is leached from the carrier by body fluids. The concentration of polypeptide in the sustained release formulation will normally be at least 1 µg/ml, usually between 10 µg/ml and 10 mg/ml. In some instances it may be desirable to continually maintain the treatment composition at the affected area or wound site during the healing period. This may be achieved via a multiplicity of intermittent applications of the treatment composition, or by administering the PDGF via a sustained release dosage form such as those described above. In this regard, the term "continually" denotes true continuous administration such as is achieved by such sustained release dosage forms or that achieved by such repeated applications that provide a pharmacokinetic pattern that mimics that achieved by true continuous administration.

### Examples

The following examples further describe the isolation of DNA encoding PDGF A-chain polypeptides and the expression of that DNA in various hosts to produce biologically active PDGF.

In the following, "digestion" refers to the enzymatic cleavage of DNA by restriction endonucleases. Restriction endonucleases commonly referred to as restriction enzymes are well characterized and commercially available and used in accordance with manufacturer's specifications. Digestion with restriction enzymes is frequently followed by treatment with alkaline phosphatase according to manufacturer's specifications to remove the terminal 5′ phosphates, thus preventing self ligation of a vector having two compatible ends.

"Fill in" refers to the enzymatic process of creating blunt ends by repairing overhanging ends generated by certain restriction enzymes. The repair is a function of DNA polymerase I large fragment (Klenow) and deoxynucleotide triphosphates and is used according to manufacturer's specifications.

Gel isolation of a DNA restriction fragment refers to the recovery of a specific fragment, electrophoretically separated on either an agarose gel or polyacrylamide gel (depending on size of fragment), by either electroelution or melting and extraction of gel slice.

All DNA manipulations are done according to standard procedures. See Maniatis et al, Molecular Cloning, Cold Spring Harbor Lab., 1982. All enzymes used are obtained from commercial sources and used according to the manufacturer's specifications.

### 1. Isolation and Characterization of PDGF A-Chain cDNA

A λgt10 cDNA library was constructed from poly (A)⁺ RNA from the human clonal glioma cell line U-343 MGaC12:6 using the LiCl/urea method modified as described by Betsholtz, C., et al, Cell (1984) 39:447-457. Oligo(dT)-primed synthesis of ds cDNA was performed according to Gubler, U., and Hoffman, B.J., Gene (1983) 25:263-299. The resulting cDNA was treated with T4 DNA polymerase and subcloned into EcoRI-cleaved λgt10 using EcoRI linkers. The recombinant phage were plated in E. coli C600hfl.

Two oligonucleotide probes, designated PDGF-A-1 and PDGF-A-2 were synthesized based on the known partial amino acid sequence of PDGF A-chain. Both were made using solid-phase phosphoramidite methodology. The double-stranded probe PDGF-A-1 was synthesized as two overlapping 50-bp oligonucleotides and radiolabeled using [α-³²P]-deoxynucleoside triphosphates and Klenow fragment of DNA polymerase I. PDGF-A-2 was synthesized as a 37-base template and a 12-base complementary primer and was radiolabeled as PDGF-A-1. The nucleotide sequences (single strand) of the two probes are given below.

PDGF-A-1 (86-mer)
CCATCGAGGAGGCCGTGCCTGCAGTGTGC
AAGAACCCGCACCGTGATCTATGAGATCCCCCGCTCC
CAGGTCGACCCCACCTCCGCC

PDGF-A-2 (37-mer)
AAGCGCTGCACCGGCTGCTGCAACACCAGCAGCGTGA

These probes were used to screen the library (2 × 10⁶ clones). Duplicate nitrocellulose filter lifts were hybridized with the probes at 42°C in 20% formamide, 5 × SSC, 50 mM sodium phosphate pH 7.0, 5 × Denhardt's, 0.10% SDS, 200 µg/ml sonicated salmon sperm DNA and washed in 0.5 × SSC, 0.1 % SDS at 42°C. Clones D1 and 13-1 were selected from among those that hybridized to both probes and sequenced by dideoxy nucleotide chain termination after subcloning into M13 phage derivatives. Partial nucleotide sequences of D1 and 13-1 are shown on Figures 1 and 2.

The longest open reading frame of D1 predicts a PDGF A-chain precursor of 211 amino acids (shown in Figure 1); the boxed portion designates the 125-amino acid PDGF A-chain polypeptide.

The deduced amino acid sequence of Figure 1 matches the reported partial sequence of the PDGF A-chain obtained by amino acid sequencing except at amino acids 119, 141, and 143, found to be Ile, Gln, and Ser, respectively, instead of the previously assigned Val, Arg, and Thr. The ATG codon at amino acid position 1 precedes a basic amino acid (Arg) followed by 18 hydrophobic residues. This is characteristic of a signal peptide sequence and is consistent with the observation that PDGF A-chain homodimers produced by human osteosarcoma cells are secreted. Comparison with preferred signal peptidase cleavage sites suggests that processing may occur between amino acids Ala20 and Glu21. The N-terminal sequence of platelet PDGF A-chain is found at amino acid 87, indicating that a propeptide of 66 amino acids (44% charged residue) is cleaved from the precursor to generate a 125-amino-acid A-chain protein. This cleavage occurs after a run of four basic amino acids, Arg-Arg-Lys-Arg. Additional proteolytic processing may occur in the C-terminal region.

The corresponding open reading frame of 13-1 (Figure 2) predicts a PDGF A-chain precursor of 196 amino-acids identical in sequence to the precursor of D1 but lacking 15 C-terminal residues. Again, the mature polypeptide is boxed in Figure 2.

### 2. Isolation of PDGF B-chain

cDNA for PDGF B-chain was isolated from the same cDNA library for use in the following experiments in which Dl, 13-1, and B-chain cDNA were cloned in an analogous manner.

### 3. Yeast Expression

Due to the ability of yeast to secrete and process proteins, the genes for the mature PDGF A-chains and B-chain were fused with the sequence of the α-factor leader, a yeast secretory signal sequence which would allow for secretion of PDGF. Yeast transformed with these plasmids would be expected to synthesize a protein containing an NH₂-terminal α-factor leader and COOH-terminal PDGF chain separated by Lys-Arg. Since this molecule is targeted for secretion, cleavage after the processing site Lys-Arg by the yeast should result in secretion of the mature growth factor. Lys-Arg is the processing site used by the natural prepro-PDGF, as well as the prepro-α-factor.

### 3.1. Regulatable Secretion in Yeast

PDGF B-chain protein, and the two forms of the A-chain protein, D1 and 13-1, are produced and secreted by yeast strain Saccharomyces cerevisiae AB110 (Mata, -ura 3-52, leu 2-04, or both leu 2-3 and leu 2-112, pep 4-3, his 4,580, cir°) transformed with yeast expression plasmids pYpNB4, pYpA6, and pYpA134 respectively. The plasmids contain the sequence coding for their respective mature PDGF protein along with pBR322 sequences including the origin of replication and the ampicillin resistance gene, as well as yeast sequences including the 2-micron and selectable markers leu and ura genes. Expression of the mature PDGF genes is under the control of the regulatable promoter ADH2-glyceraldehyde-3-phosphate-dehydrogenase (GAPDH) and α-factor terminator (see §3.1.4).

### 3.1.1. Construction of pYpNB4: A Yeast Expression Vector for PDGF B-Chain

The entire gene coding for the mature PDGF B-chain was synthesized by automated oligonucleotide synthesis on a silica support as described by Urdea et al, Proc Natl Acad Sci USA (1983) 80:7461-7465, using N,Ndiisopropyl phosphoramidites. Yeast-preferred codons were used. The synthetic gene was cloned as a 350-bp XbaI-SalI fragment into pAG (see §3.1.4), which was digested with XbaI and SalI and gel isolated to give pAGSB-4.

The expression cassette containing the synthetic PDGF B-chain gene was cut out as a BamHI fragment and cloned into the yeast shuttle vector pAB24 (see §3.1.4), previously digested with BamHI and treated with alkaline phosphatase, to give pYpNB4.

### 3.1.2. Construction of pYpA6 and pYpA134: Yeast Expression Vectors for PDGF A-Chain

In vitro mutagenesis was used to generate XbaI and SalI sites at the end of the two different mature PDGF A-chain genes in order to clone the genes into pAG (see §3.1.4). In vitro mutagenesis was performed according to the procedure of Zoller and Smith, DNA (1984) 3(6):479-488. A SacI-HindIII fragment,
containing the entire coding region for the mature polypeptide, was cloned from each of the PDGF A-chain genes into M13mp19 in order to generate single-stranded template. The following synthetic oligonucleotides were used as mutagenic primers.

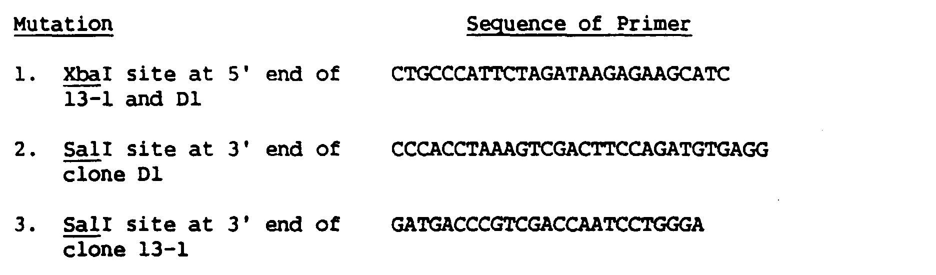

Mutagenesis of clone D1 was carried out using primers 1 and 2; primers 1 and 3 were used for clone 13-1. An approximately 400-bp XbaI-SalI (partial) fragment from clone D1 and an approximately 360-bp XbaI-SalI (partial) fragment from clone 13-1 were each isolated and cloned into pAG, which was digested with the XbaI and SalI and gel isolated, to give pAG-AD1 and pAGA13.1, respectively. The expression cassettes containing the two forms of the mature PDGF A-chain gene were cut out as BamHI fragments and each cloned into pAB24, previously digested with BamHI and treated with alkaline phosphatase, to give plasmids pYpA6 and pYpA134.

### 3.1.3. Yeast Transformation and Expression

Yeast expression plasmids pYpNB4, pfpA6, and pYp134 were transformed into into yeast strain S. cerevisiae AB110 (Mata, ura 3-52, leu 2-04, or both leu 2-3 and leu 2-l12, pep 4-3, his 4-580**,** cir°), as described by Hinnen et al (Proc Natl Acad Sci USA (1978) 75:1929-1933) and plated on ura⁻, 8% glucose, sorbitol plates. Transformants are grown in leu⁻, 8% glucose liquid medium for 24 hr and then plated onto leu⁻, 8% glucose sorbitol plates to get individual colonies. Individual colonies are picked and grown in 3 ml of leu, 8% glucose medium for 24 hr at 30°C, and then inoculated (1:50) into 1 liter of ura⁻, 1% glucose media and grown for 75 hr at 30°C. Yeast culture medium was assayed for PDGF activity by the human foreskin fibroblast mitogen assay (see §5). The yeast transformant pYpNB4-2 secretes PDGF B-chain into the medium at a level of 500 ng/ml, transformant pYpA6-NT1 secretes PDGF A-chain (D1 form) into the medium at a level of 750 ng/ml and transformant pYpA134-NT1 secretes PDGF A-chain (13-1 form) into the medium at a level of 325 ng/ml.

Proteins from the above cultures were run on a 15% polyacrylamide SDS gel. The B-chain migrates at the expected size of 14.5 Kd. The 13.1 form of A-chain migrates as 2 bands at 18 and 18.5 Kd respectively. The size for the expected single species is 18 Kd. The D1 form of A-chain migrates as 3 bands at 19, 18.5 and 18 Kd. Its expected size on the same gel for a single species is calculated at 19.5 Kd. The extra bands for both A-chains are due to proteolytic cleavage at either the amino-terminus and/or the carboxy-terminus.

### 3.1.4. Piasmids Used for The Preparation of Yeast Expression Vectors

pAG is a general yeast expression cassette vector derived from pAG-TNF where pAG-TNF is only used for convenience of cloning with the pAG construct being relevant to this application.

The expression cassette vector, pAG-TNF, contains the regulatable ADH2-GAPDH promoter, the α-factor leader, the synthetic TNF gene, and the α-factor terminator cloned in pBRΔRI-Sal. The ADH2GAPDH promoter was isolated as a 1.0-Kbp BamHI-NcoI fragment from pAGΔXbaGAP1. The 5' end of the α-factor leader was supplied as a synthetic adaptor for the following sequence and having NcoI- and PstI-compatible overhangs: 
CATGAGATTCCTTCAATTTTTACTGCA
TCTAAGGAAGTTAAAAATG
 The 3' end of the α-factor leader, the synthetic TNF gene and the α-factor terminator was isolated as a PstI-BamHI fragment from pHG100-TNF. The three fragments were ligated together and cloned into pBRΔRI-Sal which had been previously digested with BamHI and treated with alkaline phosphatase.

pBRΔRI-Sal was constructed by digesting pBR322 with EcoRI and SalI, filling in the overhangs with Klenow fragment, and ligating on BamHI linkers. The vector and linkers were digested with BamHI and the BamHI-BamHI 3.8 kb vector was gel isolated and recircularized by self-ligation. The resulting plasmid was designated pBRΔRI-Sal.

The plasmid pAGΔXbaGAP1 contains the ADH2-GAPDH hybrid promoter and the GAPDH terminator cloned into pBRΔRI-Sal. The ADH2-GAPDH promoter (the only sequence pertinent to this application) was isolated as a 1.1-kb BamHI-NcoI fragment from pJS103 (described below). In addition, an approximately 90-bp XbaI-XbaI deletion was introduced into the 5' end of the promoter fragment by cutting the plasmid with XbaI, filling in the overhang ends with Klenow fragment, and dNTPs and recircularizing the plasmid, thus giving pAGΔXbaGAP1.

pHG100-TNF contains the α-factor promoter, leader, and terminator with the synthetic gene coding for TNF inserted in frame at the 3' end of the leader. The 1.0 kb Pst-BamHI fragment isolated from this plasmid contains 240 bp of the 3' end of the α-factor leader, the 494-bp synthetic TNF gene (as an XbaI-SalI fragment) and the 272-bp α-factor terminator. The α-factor sequences which are the only sequences relevant to this application are derived from pAB114. pAB114 is described in EPO 0 116 201, pages 14-18, and Brake, A.J., et al, Proc Natl Acad Sci USA (1984), 81:4642-4646. The only difference is that a silent mutation was introduced by M13 mutagenesis to create an XbaI site at the 3' end of the leader to facilitate cloning of heterologous genes.

The comparison of the 3' end of the α-factor leader from the wild type (pAB114) versus the altered α-factor (pHG100) illustrated below shows that a silent mutation was incorporated to code for an XbaI site just 5' to the processing site (LysArg). This allows for insertion of heterologous genes without the "spacer" codons (must provide the LysArg processing site and maintain reading frame).

α-factor leader    processing site    spacer    α-factor gene
IleAlaAlaLysGluGluGlyValSerLeuAspLysArgGluAlaGluAlaTrpHisTrpLeuGlnLeuGly
pAB114    ATTGCTGCTAAAGAAGAAGGGGTATCTTTGGATAAAAGAGAGGCTGAAGCTTGGCATTGGTTGCAACTGGGG
XbaI    HindIII
pHG100    ATTGCTGCTAAAGAAGAAGGGGTATCTCTAGATAAAAGAGCTCCAACCTCTTCCTCTACCAAGAAGACCCAG
IleAlaAlaLysGluGluGlyValSerLeuAspLysArgAlaProThrSerSerSerThrLysLysThrGln
α-factor leader    processing site    gene for IL-2

### pJS103

Plasmid pJS103 contains the inducible hybrid ADH2-GAPDH promoter. The hybrid promoter is made up of the transcriptional and translational initiation region from the GAPDH promoter and is under the regulatory control of the ADH2 transcriptional regulatory region. The ADH2 transcriptional regulatory region is derepressed in the absence of a readily available source such as glucose (without exogenous inducer). By allowing for glucose exhaustion after the yeast culture is grown to high density, the transcriptional control region will be derepressed and expression of the desired peptide will occur.

Plasmid pJS103 was constructed as follows. The ADH2 portion of the promoter was constructed by cutting a plasmid containing the wild-type ADH2 gene from plasmid pADR2 (Beier et al, Nature (1982) 300:724-728) with restriction enzyme EcoRV, which cuts at position +66 relative to the ATG start codon, as well as in two other sites in pADR2, outside of the ADH2 region. The resulting mixture of a vector fragment and two smaller fragments was resected with Bal31 exonuclease to remove about 300 bp. Synthetic XhoI linkers were ligated onto the Bal31-treated DNA. The resulting DNA linker vector fragment (about 5 kb) was separated from the linkers by column chromatography, cut with restriction enzyme XhoI, religated, and used to transform E. coli to ampicillin resistance. The positions of the XhoI linker were determined by DNA sequencing. One plasmid which contained an XhoI linker within the 5' nontranscribed region of the ADH2 gene (position -232 from ATG) was cut with the restriction enzyme XhoI, treated with nuclease S1, and subsequently treated with the restriction enzyme EcoRI to create a linear vector molecule having one blunt end at the site of the XhoI linker and an EcoRI end. The GAP portion of the promoter was constructed by cutting plasmid pPGAP1 with the enzymes BamHI and EcoRI, followed by the isolation of the 0.4 Kbp DNA fragment. This purified fragment was then completely digested with the enzyme AluI and an approximately 200 bp fragment was isolated.

This GAP promoter fragment was ligated to the ADH2 fragment present on the linear vector described above to give plasmid pJS103.

### pPGAP1

pPGAP1 is a yeast expression cassette vector which has a polyrestriction site linker between the GAPDH terminator and a truncated GAPDH promoter region. The polyrestriction site contains the recognition sites for NcoI, EcoRI, and SalI, and the cassette is excisable as a BamHI fragment. The preparation of pPGAP1 is described in EPO 0 164 556 and Travis, J., et al, J Biol Chem (1985) 260(7):4384-4389. In both references pPGAP1 is referred to pPGAP.

### pAB24

pAB24 is a yeast shuttle vector which contains the complete 2 µ sequences (Broach, In: Molecular Biology of the Yeast Saccharomyces, 1:445, Cold Spring Harbor Press (1981)) and pBR322 sequences. It also contains the yeast URA3 gene derived from plasmid YEp24 (Botstein et al, Gene (1979) 8:17) and the yeast LEU2^{d} gene derived from plasmid pCl/1 (described in European Patent Application publication no. EPO116201). Insertion of the expression cassette was in the BamHI site of pBR322, thus interrupting the gene for bacterial resistance to tetracycline.

### 3.2. Intracellular Expression in Yeast

PDGF B-chain and the two forms of the A-chain protein, D1 and 13.1, can be produced internally by yeast.strain S. cerevisiae AB110 transformed with pAB24-AGMetPDGF-SB, pAB24-AGMetPDGF-AD1, and pAB24-AGMetPDGF-A13.1 respectively. The plasmids contain the sequence coding for their respective mature PDGF protein with an additional methionine at the amino terminus which allows for direct expression. Expression of the mature PDGF genes is under the control of the regulatable promoter ADH2-GAPDH and the GAPDH terminator.

### 3.2.1. Construction of PAB24-AGMetPDGF-SB: A Yeast Expression Vector for PDGF B-Chain

In order to express the mature B-chain internally in yeast, plasmid pAGSB-4 (§3.1.1) is digested with BglII and then treated with S1 nuclease to remove the 5' overhang and generate a blunt end. A linker of the following sequence: 
5'-GATCTATGTC -3'
       ATACAG
 (which contains a BglII overhang, a start codon, and regenerates the codon coding for the first amino acid, serine, of the mature PDGF B-chain) is ligated to pAGSB4 and then the plasmid is digested with BglII and SalI. The 345 bp BglII-SalI fragment is gel isolated and ligated into BglII and XhoI digested pRSP101 to generate plasmid pAB24-AGMetPDGF-SB.

The plasmid pRSP101 is a yeast expression vector containing the regulatable ADH2-GAPDH promoter and GAPDH terminator and was derived from pAB24 and pBS100. Plasmid pRSP100 was constructed by excising the BamHI cassette from pBS100 and ligating it into pAB24 which had been previously digested with BamHI and treated with phosphatase. The intermediate vector was then digested with NcoI and SalI, to remove the fragment between the promoter and the terminator, and then treated with S1 nuclease to remove the single-stranded overhangs and make the ends blunt. A linker of the following sequence: 
5'-AGATCTCTTGCTCGAG -3'
TCTAGAGAACGAGCTC
 was then ligated in to add unique sites for BglII and XhoI. Plasmid pBS100 is a yeast expression cassette vector cloned into a pBR322 derivative pAB12. The expression cassette contains the hybrid ADH2-GAPDH promoter and the GAPDH terminator flanking a gene segment from the HIV envelope gene. The ADH2-GAPDH promoter is a 1200 bp BamHI-NcoI fragment isolated from pJS103 (§3.1.4) and the GAPDH terminator is a 900 bp SalI-BamHI fragment isolated from plasmid pPGAP1 (§3.1.4). Plasmid pBS100 also contains a non-essential fragment between the NcoI and SalI sites which is replaced by gene fragments of interest. The expression cassette can be removed from pBS100 by digestion with BamHI and cloned into yeast shuttle vectors for introduction into yeast cells.

Plasmid pAB12 is a pBR322 derivative lacking the region between the single HindIII and SalI sites and containing a BamHI linker inserted between the unique EcoRI site. This vector was constructed by digesting pBR322 to completion with HindIII and SalI followed by limited digestion wtih Bal31 nuclease, repair of the ends so created with the Klenow fragment of E. coli DNA polymerase I, and blunt-end ligation with T4 DNA ligase to reform closed covalent circles. The plasmid was then opened up with EcoRI, treated with the Klenow fragment of E. coli DNA polymerase I (to fill-in the 5' overhangs), blunt-end ligated with BamHI linkers, digested with BamHI to remove excess linkers, and then ligated to form closed circles.

### 3.2.2. Construction of pAB24-AGMetPDGF-AD1 and pAB24-AGMetPDGF-A13.1: Yeast Expression Vectors for PDGF A-Chain

In order to clone the two mature A-chains of PDGF, to be expressed internally in yeast, the semisynthetic NcoI-HindIII fragments are isolated from the internal bacterial expression vectors pSOD-MethPDGF-AD1 and pSOD-MethPDGF-A13.1 (§4.2). These fragments contain the translational start codon ATG followed by the mature PDGF A-chain gene and the 3' untranslated region. The approximately 613 bp and 544 bp NcoI-HindIII fragments coding for D1 and 13.1 respectively are each ligated into pBS100 which is previously digested with NcoI and HindIII (cuts in the 5' end of the terminator region) to give the resulting plasmids pAGMetPDGF-AD1 and pAGMetPDGF-A13.1. The expression cassette containing the ADH2-GAPDH hybrid promoter, mature PDGF A-chain gene, and the GAPDH terminator is excised as a BamHI fragment and ligated into pAB24 which is previously digested with BamHI and treated with phosphatase to yield plasmids pAB24AGMetPDGF-AD1 and pAB24 AGMetPDGF-A13.1.

### 3.2.3. Yeast Transformation and Expression

The yeast expression plasmids containing MetPDGF B-chain and two forms of A-chain: pAB24-AGMetPDGF-SB, pAB24-AGMetPDGF-AD1, and pAB24-AGMetPDGF-A13.1, were each transformed into yeast S. cerevisiae AB110 as described previously in §3.1.3.

### 3.3. Internal Expression as an SOD-Fusion

### 3.3.1. Construction of pAB24-AGhSOD-hPDGF: A Yeast Expression Vector for an SOD-PDGF B-Chain Fusion Protein

To make the above plasmid, the BglII-SalI fragment encoding the mature PDGF B-chain is excised from pYpNB4. The following linker is ligated onto the 5' end: 
5'-AATTCTAAAA
      GATTTTCTAG-3'
 This linker includes a lys arg cleavage site for the Kex2 protease to allow separation of human superoxide dismutase (hSOD) and PDGF B-chain. The EcoRI-SalI fragment is then cloned into phosphatase-treated pSI4 (described in European Patent Application 86104066.5, publication no 0196056) digested with EcoRI and SalI. The BamHI cassette is then excised and subcloned into phosphatase-treated pAB24 digested with BamHI.

### 3.3.2. Construction of pAB24-AGSOD-hPDGF A (13.1 or D1):

### A Yeast Expression Vector for an SOD-PDGFA (13.1 or D1) Fusion Protein

To make the above plasmid, the SalI fragments containing 102 and 87 of the N-terminal amino acids of mature PDGF A-chain forms D1 and 13.1 respectively are isolated. They are ligated to linkers that restore the first 23 amino acids of PDGF A-chain, introduce a lys arg (Kex2) cleavage site between SOD and PDGF, and an EcoRI restriction site at the 5' end.

5' AAT TCT AAA AGA TCT (sequence for next G
GA TTT TCT AGA 22 aa of CAG CG
PDGF A-chain)

EcoRI          Lys Arg Ser                              SalI
 site               (lst aa in                          site
                     PDGF A-chain)

The EcoRI-SalI fragment (355 and 392 bp for clones 13.1 and D1 respectively) are then ligated into phosphatase treated pSI4 digested with EcoRI and SalI. The BamHI cassette is excised and subcloned into phosphatase-treated pAB24 digested with BamHI.

### 4. E. coli : Vectors used in production of yeast vectors

### 4.1. Construction of pSOD-MethPDGF-SB: A Bacterial Expression Vector for PDGF B-Chain

The entire coding region for the PDGF B-chain was synthesized as described previously (§3.1.1) except that an NcoI site was generated at the 5' end instead of the XbaI site. The 350 bp NcoI-SalI PDGF B fragment was ligated into pSODCF2, digested previously with NcoI and SalI and gel isolated, to give plasmid pSOD-MethPDGF-SB.

pSODCF2 is an E. coli expression vector containing the TacI promoter (a hybrid trp-lac promoter) followed by a cDNA copy of the hSOD gene and a polylinker (for cloning COOH terminal fusions) cloned into pBR322. pSODCF2 is described in Steimer, K.S., et al, J Virol (1986) 58:9-16.

### 4.2. Construction of pSOD-MethPDGF-ADl and pSOD-MethPDGF-A13.1: Bacterial Expression Vectors for PDGF A-Chain

In order to clone the two forms of the mature PDGF A-chain for E. coli expression the first 69 nucleotides were synthesized to include an NcoI site and code for a methionine at the 5' end and a SalI site at the 3' end. This fragment was ligated with each of the approximately 475-bp SalI-HindIII fragment from clone 13-1 and the approximately 545 bp SalI-HindIII fragment from clone D1.

The resulting semisynthetic genes for both of the A-chains were cloned as NcoI-HindIII fragments into pbaFGF_{Fix} which was previously digested with NcoI and HindIII and gel isolated to give pSOD-MethPDGF-AD1 and pSOD-MethPDGF-A13.1, respectively.

The plasmid pbaFGF_{Fix} is plasmid pSODCF2 containing a 436-bp NcoI-SalI fragment coding for fibroblast growth factor (FGF) and was used only for convenience due to the presence of a HindIII site within the FGF sequences. Translation stop codons for PDGF are present, as evidenced by the sequence.

### 5. Bioassay for PDGF Activity

### 5.1 PDGF Produced in Yeast

Samples of supernatants of yeast which had expressed PDGF were appropriately diluted, depending upon expected activity, and then serially diluted in DMEM containing 1% bovine serum albumin (BSA). Aliquots (10µl) of each dilution were placed in the wells of the assay plates.

### 5.2 Human Foreskin Fibroblast (HFF) Mitogen Assay for PDGF

HFF stocks were stored frozen; freezing was at passage 13. Prior to use, HFF were thawed, and grown in T75 flasks until confluent, which usually occurred at 5-7 days. Growth medium contained Dulbecco's Modified Eagles Medium (DMEM), 20% fetal bovine serum (FBS), 1 mM sodium pyruvate, 300 µg/ml L-glutamine, 100U/ml penicillin, and 100 µg/ml streptomycin. Cells were incubated at 37°C in humidified 7% CO₂, 93% air atmosphere. At confluency, cells were passaged by rinsing the monolayer with phosphate buffered saline (PBS) lacking Ca⁺⁺ and Mg⁺⁺, dissociating them in trypsin containing EDTA, and diluting them with Growth Medium. Cells were passaged no more than 8 times after thawing.

To assay for PDGF, HFFs were plated as follows. The cells were rinsed and dissociated with trypsin as above. The trypsinized cells were pelleted, and resuspended to a concentration of 1 x 10⁵ cells/ml in medium similar to Growth Medium, except that 5% FBS replaced 20% FBS; 100 µl of suspension was dispensed into each well of a 96 well microtiter plate, and the cells were incubated 5-6 days under the above described conditions.

PDGF in the sample was determined by monitoring ³H-thymidine incorporation into HFF DNA stimulated by PDGF. Samples were added to the wells containing HFF monolayers, and the assay plates incubated as above for 18 hours. The HFF cultures were then pulsed with [Methyl³H] thymidine (10 µC/ml final concentration, 1 µC/well) at 37°C under the above described incubation conditions for 8 hours. After incubation, the cells were rinsed with PBS and fixed. Fixing was by incubation with 5% trichloracetic acid (TCA) and then 100% methanol for 15 minutes, followed by drying in air. The cells were then solubilized with 0.3N NaOH, and counted in a liquid scintillation counter.

Control samples were treated as the samples described above, and were prepared as follows. For positive controls, PDGF, purchased from PDGF, Inc., was dissolved to a final concentration of 100 ng/ml in DMEM containing 10 mg/ml BSA. A standard curve was prepared; the first point was 10 ng/ml, the remaining points were 2-fold serial dilutions. Each dilution was tested in triplicate. Negative controls, which lacked both sample and control PDGF, were also run.

Modifications of the above-described modes for carrying out the invention that are obvious to those of skill in the fields related to the invention are intended to be within the scope of the following claims.

## Claims (Claims for the following Contracting State(s): AT, BE, CH, DE, FR, GB, IT, LI, LU, NL, SE)

1. A recombinant yeast expression vector comprising:
(i) a DNA sequence which encodes a PDGF A-chain polypeptide comprising the amino acid sequence numbered 87 to 193, inclusive, in Figure 1;
(ii) a DNA sequence which encodes a PDGF A-chain polypeptide comprising the amino acid sequence numbered 87 to 196, inclusive, in Figure 1 or Figure 2;
(iii) a DNA sequence which encodes a PDGF A-chain polypeptide comprising the amino acid sequence numbered 87 to 211, inclusive, in Figure 1;
(iv) a DNA sequence which encodes a PDGF A-chain polypeptide comprising the amino acid sequence numbered 1 to 196, inclusive, in Figure 1 or Figure 2; or
(v) a DNA sequence which encodes a PDGF A-chain polypeptide comprising the amino acid sequence numbered 1 to 211, inclusive, in Figure 1;
operably linked to a yeast hybrid ADH-GAPDH promoter.

2. A yeast expression vector according to claim 1 which further comprises a DNA sequence encoding human superoxide dismutase between the promoter and PDGF A-chain sequence.

3. A yeast expression vector according to claim 1 which comprises a pYpA6 as shown in Figure 3; pYpA134 as shown in Figure 4; pAB24-AGMetPDGF-AD1 or pAB24-AGMetPDGF-A13 as shown in Figure 5 or pAB24-AGSOD-hPDGFAD1 or pAB24-AGSOD-hPDGFA13.1 as shown in Figure 6.

4. Yeast cells transformed with the recombinant expression vector of claim 1, 2 or 3.

5. A method of producing recombinant PDGF comprises of PDGF A-chain polypeptide, said method comprising growing the yeasts cells of claim 4.

## Claims (Claims for the following Contracting State(s): ES, GR)

1. A method of producing recombinant PDGF comprises of PDGF A-chain polypeptide, said method comprising growing yeast cells transformed with a recombinant yeast expression vector comprising:
(i) a DNA sequence which encodes a PDGF A-chain polypeptide comprising the amino acid sequence numbered 87 to 193, inclusive, in Figure 1;
(ii) a DNA sequence which encodes a PDGF A-chain polypeptide comprising the amino acid sequence numbered 87 to 196, inclusive, in Figure 1 or Figure 2;
(iii) a DNA sequence which encodes a PDGF A-chain polypeptide comprising the amino acid sequence numbered 87 to 211, inclusive, in Figure 1;
(iv) a DNA sequence which encodes a PDGF A-chain polypeptide comprising the amino acid sequence numbered 1 to 196, inclusive, in Figure 1 or Figure 2; or
(v) a DNA sequence which encodes a PDGF A-chain polypeptide comprising the amino acid sequence numbered 1 to 211, inclusive, in Figure 1;
operably linked to a yeast hybrid ADH--GAPDH promoter.

2. A method according to claim 1 where the yeast expression vector further comprises a DNA sequence encoding human superoxide dismutase between the promoter and PDGF A-chain sequence.

3. A method according to claim 1 where the yeast expression vector comprises pYpA6 as shown in Figure 3; pYpA134 as shown in Figure 4 ; pAB24-AGMetPDGF-AD1 or pAB24-AGMetPDGF-A13 as shown in Figure 5 or pAB24-AGSOD-hPDGFAD1 or pAB24-AGSOD-hPDGFA13.1 as shown in Figure 6.

## Patentansprüche (Patentansprüche für folgende(n) Vertragsstaat(en): AT, BE, CH, DE, FR, GB, IT, LI, LU, NL, SE)

1. Rekombinanter Hefeexpressionsvektor, umfassend:
(i) eine DNA-Sequenz, die für ein PDGF-A-Kettenpolypeptid codiert, welches die Aminosäuresequenz enthält, die 87 bis einschließlich 193 in Figur 1 numeriert ist;
(ii) eine DNA-Sequenz, die für ein PDGF-A-Kettenpolypeptid codiert, welches die Aminosäuresequenz enthält, die 87 bis einschließlich 196 in Figur 1 oder Figur 2 numeriert ist;
(iii) eine DNA-Sequenz, die für ein PDGF-A-Kettenpolypeptid codiert, welches die Aminosäuresequenz enthält, die 87 bis einschließlich 211 in Figur 1 numeriert ist;
(iv) eine DNA-Sequenz, die für ein PDGF-A-Kettenpolypeptid codiert, welches die Aminosäuresequenz enthält, die 1 bis einschließlich 196 in Figur 1 oder Figur 2 numeriert ist; oder
(v) eine DNA-Sequenz, die für ein PDGF-A-Kettenpolypeptid codiert, welches die Aminosäuresequenz enthält, die 1 bis einschließlich 211 in Figur 1 numeriert ist;
operierbar an einen Hefehybrid-ADH-GAPDH-Promotor gekoppelt.

2. Hefeexpressionsvektor nach Anspruch 1, enthaltend weiter eine DNA-Sequenz, die für humane Superoxid-Dismutase codiert, zwischen dem Promotor und der PDGF-A-Kettensequenz.

3. Hefeexpresssionsvektor nach Anspruch 1, welcher ein pYpA6, wie in Figur 3 dargestellt, pYpA134, wie in Figur 4 dargestellt, pAB24-AGMetPDGF-AD1 oder pAB24-AGMetPDGF-A13, wie in Figur 5 dargestellt, oder pAB24-AGSOD-hPDGFAD1 oder pAB24-AGSOD-hPDGFA13.1, wie in Figur 6 dargestellt, umfaßt.

4. Hefezellen, transformiert mit dem rekombinanten Expressionsvektor nach Anspruch 1, 2 oder 3.

5. Verfahren zur Herstellung eines rekombinanten PDGF, umfassend ein PDGF-A-Kettenpolypeptid, wobei das Verfahren das Züchten der Hefezellen nach Anspruch 4 umfaßt.

## Patentansprüche (Patentansprüche für folgende(n) Vertragsstaat(en): ES, GR)

1. Verfahren zur Herstellung eines rekombinanten PDGF, enthaltend ein PDGF-A-Kettenpolypeptid, wobei das Verfahren das Züchten von Hefezellen umfaßt, die mit einem rekombinanten Hefeexpressionsvektor transformiert sind, der umfaßt:
(i) eine DNA-Sequenz, die für ein PDGF-A-Kettenpolypeptid codiert, welches die Aminosäuresequenz enthält, die 87 bis einschließlich 193 in Figur 1 numeriert ist;
(ii) eine DNA-Sequenz, die für ein PDGF-A-Kettenpolypeptid codiert, welches die Aminosäuresequenz enthält, die 87 bis einschließlich 196 in Figur 1 oder Figur 2 numeriert ist;
(iii) eine DNA-Sequenz, die für ein PDGF-A-Kettenpolypeptid codiert, welches die Aminosäuresequenz enthält, die 87 bis einschließlich 211 in Figur 1 numeriert ist;
(iv) eine DNA-Sequenz, die für ein PDGF-A-Kettenpolypeptid codiert, welches die Aminosäuresequenz enthält, die 1 bis einschließlich 196 in Figur 1 oder Figur 2 numeriert ist; oder
(v) eine DNA-Sequenz, die für ein PDGF-A-Kettenpolypeptid codiert, welches die Aminosäuresequenz enthält, die 1 bis einschließlich 211 in Figur 1 numeriert ist;
operierbar an einen Hefehybrid-ADH-GAPDH-Promotor gekoppelt.

2. Verfahren nach Anspruch 1, wobei der Hefeexpressionsvektor weiter eine DNA-Sequenz, welche für humane Superoxid-Dismutase codiert, zwischen dem Promotor und der PDGF-A-Kettensequenz, umfaßt.

3. Verfahren nach Anspruch 1, wobei der Hefeexpressionsvektor pYpA6, wie in Figur 3 dargestellt, pYpA134, wie in Figur 4 dargestellt, pAB24-AGMetPDGF-AD1 oder pAB24-AGMetPDGF-A13, wie in Figur 5 dargestellt, oder pAB24-AGSOD-hPDGFAD1 oder pAB24-AGSOD-hPDGFA13.1, wie in Figur 6 dargestellt, umfaßt.

## Revendications (Revendications pour l'(les) Etat(s) contractant(s) suivant(s): AT, BE, CH, DE, FR, GB, IT, LI, LU, NL, SE)

1. Vecteur d'expression de levure recombiné comprenant:
(i) une séquence d'ADN qui code pour un polypeptide de la chaîne A de PDGF comprenant la séquence d'acides aminés numérotés 87 à 193, inclus, dans la figure 1;
(ii) une séquence d'ADN qui code pour un polypeptide de la chaîne A de PDGF comprenant la séquence d'acides aminés numérotés 87 à 196, inclus, dans la figure 1 ou la figure 2;
(iii) une séquence d'ADN qui code pour un polypeptide de la chaîne A de PDGF comprenant la séquence d'acides aminés numérotés 87 à 211, inclus, dans la figure 1;
(iv) une séquence d'ADN qui code pour un polypeptide de la chaîne A de PDGF comprenant la séquence d'acides aminés numérotés 1 à 196, inclus, dans la figure 1 ou la figure 2; ou
(v) une séquence d'ADN qui code pour un polypeptide de la chaîne A de PDGF comprenant la séquence d'acides aminés numérotés 1 à 211, inclus, dans la figure 1;
liée de manière opérationnelle à un promoteur hybride d'ADH-GAPDH de levure.

2. Vecteur d'expression de levure selon la revendication 1, qui comprend de plus une séquence d'ADN codant pour une superoxyde dismutase humaine entre le promoteur et la séquence de la chaîne A de PDGF.

3. Vecteur d'expression de levure selon la revendication 1, qui comprend un pYpA6 comme représenté à la figure 3; un pYpA134 comme représenté à la figure 4; un pAB24-AGMetPDGF-AD1 ou un pAB24-AGMetPDGF-A13 comme représenté à la figure 5 ou un pAB24-AGSOD-hPDGFAD1 ou un pAB24-AGSOD-hPDGFA13.1 comme représenté à la figure 6.

4. Cellules de levure transformées par le vecteur d'expression recombiné de la revendication 1, 2 ou 3.

5. Procédé de production d'un PDGF recombiné qui comprend un polypeptide de la chaîne A de PDGF, ledit procédé comprenant la croissance des cellules de levures de la revendication 4.

## Revendications (Revendications pour l'(les) Etat(s) contractant(s) suivant(s): ES, GR)

1. Procédé de production d'un PDGF recombiné qui comprend un polypeptide de la chaîne A de PDGF, ledit procédé comprenant la croissance de cellules de levure transformées par un vecteur d'expression de levure recombiné comprenant :
(i) une séquence d'ADN qui code pour un polypeptide de la chaîne A de PDGF comprenant la séquence d'acides aminés numérotés 87 à 193, inclus, dans la figure 1;
(ii) une séquence d'ADN qui code pour un polypeptide de la chaîne A de PDGF comprenant la séquence d'acides aminés numérotés 87 à 196, inclus, dans la figure 1 ou la figure 2;
(iii) une séquence d'ADN qui code pour un polypeptide de la chaîne A de PDGF comprenant la séquence d'acides aminés numérotés 87 à 211, inclus, dans la figure 1;
(iv) une séquence d'ADN qui code pour un polypeptide de la chaîne A de PDGF comprenant la séquence d'acides aminés numérotés 1 à 196, inclus, dans la figure 1 ou la figure 2; ou
(v) une séquence d'ADN qui code pour un polypeptide de la chaîne A de PDGF comprenant la séquence d'acides aminés numérotés 1 à 211, inclus, dans la figure 1;
liée de manière opérationnelle à un promoteur hybride d'ADH-GAPDH de levure.

2. Procédé selon la revendication 1, dans lequel le vecteur d'expression de levure comprend de plus une séquence d'ADN codant pour une superoxyde dismutase humaine entre le promoteur et la séquence de la chaîne A de PDGF.

3. Procédé selon la revendication 1, dans lequel le vecteur d'expression de levure comprend pYpA6 comme représenté à la figure 3; pYpA134 comme représenté à la figure 4; pAB24-AGMetPDGF-ADI ou pAB24-AGMetPDGF-A13 comme représenté à la figure 5 ou pAB24-AGSOD-hPDGFAD1 ou pAB24-AGSOD-hPDGFA13.1 comme représenté à la figure 6.
